# EUROPEAN PATENT SPECIFICATION

(11) **EP 1 018 953 B1**
(45) Date of publication and mention of the grant of the patent: **28.12.2005**
(21) Application number: 98950808.0
(22) Date of filing: 30.09.1998
(51) Int. Cl.: A61B 17/22

(54) **BASKET WITH ONE OR MORE MOVEABLE LEGS**
FANGKÖRBCHEN MIT EINEM ODER MEHREREN BEWEGLICHEN GREIFARMEN
PANIER COMPRENANT UN OU PLUSIEURS BRAS MOBILES

(30) Priority: 01.10.1997 US 60821 P; 23.04.1998 US 65158
(43) Date of publication of application: 19.07.2000
(73) Proprietor: Boston Scientific Limited, St. Michael (BB)
(72) Inventor: BATES, James, S., Bloomington, IN 47429 (US); WARD, Tim, E., Ellettsville, IN 47429 (US)
(74) Representative: Lawrence, John
(86) International application number: PCT/US1998/020556
(87) International publication number: WO 1999/016364

(56) References cited:
- WO-A-92/16153
- DE-A- 2 804 058
- DE-A- 3 522 649
- DE-A- 3 633 527
- DE-U- 8 707 515
- FR-A- 2 694 687
- US-A- 3 739 784
- US-A- 4 807 626

## Description

### Technical Field

The invention generally relates to medical retrieval devices for retrieving material from within a body. More particularly, the invention relates to such a device that has a basket at its distal end with one or more legs that are actuateable independent of the other legs for maneuvering the basket around the material (e.g., a stone of some kind) to enhance the basket's ability to capture the material.

### Background Information

Baskets are used to retrieve biological material from the body. Baskets are used, for example, to retrieve stones from the urinary tract (e.g., ureteral stones) or stones from the biliary tree (e.g., bile duct stones). Baskets may or may not be used through a catheter, an endoscope, or a laparoscope.

Existing retrieval baskets generally consist of legs, and the diameter and overall shape of the basket is defined by the number, flexibility, shape, and length of the legs. The legs generally are equal and fixed in length, providing overall symmetry to the basket shape. The legs of the basket typically are joined at the tip of the basket and at the base of the basket closest to the sheath. The legs can be joined with solder, by welding the legs together, or by some type of mechanical mechanism. At the basket base, the joined legs typically also are attached to a shaft, wire, or coil. The shaft can be moved back and forth within the sheath or catheter by an actuation device such as a proximal handle with a back-and-forth hand-activated slider. Alternatively, the sheath can be moveable back and forth to expose and cover the basket. In any case, the basket is exposed and expanded fully, within the sheath and collapsed fully, or somewhere in between those two extreme positions, and the legs generally all move in a collective manner as they are joined at both the tip and base to form the basket.

US Patent No. 4 198 960 to Mikio Utsugi discloses an apparatus for removing foreign matter from the human body cavity. The apparatus includes a sheath, a central channel, a connecting member, and a plurality of trapping wires. The trapping wires are individually moveable lengthwise of the sheath and have ends connected to the connecting member.

### Summary of the Invention

The invention features a medical retrieval device as defined in claim 1. It has a basket with one or more legs that are actuateable or moveable independent from the other legs to facilitate the capture of material (e.g., a stone) from within the body and to improve retention of the captured material in the basket. For example, one leg of a four leg basket can be independently moveable, or two of the four legs can be actuated together independent of the other two legs, or three legs of a five leg basket can be moveable together independent of the fourth and fifth legs. Baskets according to the invention can be tilted, maneuvered, and/or steered to achieve capture and release of the material. A basket of the invention also allows improved dilation or opening force to expand a tract in which the basket is placed. According to the invention, the shape and size of the distance between at least one pair of basket legs is adjustable by individual action of one or more legs. That is, when the basket is maneuvered to capture a stone, at least one leg can be actuated independently to adjust the distance between the basket legs. Once the stone is captured, the independently actuateable leg(s) can be moved again alone and/or with the other legs to retain the stone. In one embodiment, the distal end of the basket is non-perforated, which can aid in retaining the captured material within the basket.

At least one of the legs is coupled to a first elongate member extending within the lumen of the sheath to the handle, and at least one other different one of the legs can be coupled to a second elongate member extending within the lumen of the sheath to the handle, such that independent movement of the first elongate member within the sheath causes independent movement of the leg coupled thereto and independent movement of the second elongate member within the sheath causes independent movement of the leg coupled thereto. Also, at least one of the legs can include an inner surface which has at least a portion that is roughened. The basket shape can have a distal end which is non-perforated.

In a preferred embodiment the invention involves a device of the type described above with a handle, sheath and basket. This device also includes first and second elongate members, a ring, and a stop. The first elongate member is disposed and moveable within the lumen of the sheath, and it is attached at one end to a base of the basket. The ring encircles the first elongate member, and it is disposed and moveable within the lumen of the sheath. The second elongate member is moveable within the lumen of the sheath, and it is attached at one end to the ring and at the other end to at least one of the legs. The stop is located within the lumen of the sheath for contact by the ring to prevent the second elongate member from advancing beyond a predetermined distance within the lumen such that further movement by the first elongate member within the lumen toward the distal end of the sheath causes the basket to "mushroom" or otherwise alter its shape.

The basket shape has a distal end which is non-perforated, and at least one of the legs is moveable independently from at least one of the other legs.

Embodiments according to another preferred embodiment of the invention can include various features. For example, each of the legs can be coupled to an elongate member extending within each of the lumens to the handle, and at least one of the elongate members can be moveable independently from at least one of the other elongate members, such that independent movement of at least one of the elongate members within the sheath causes independent movement of the leg coupled thereto. Also, at least one of the legs can include an inner surface which has at least a portion that is roughened to facilitate purchase on the item to be retrieved.

The device of the invention can be used in a method for retrieving material from a body. The method comprises inserting a medical retrieval device (such as a device described above) into a body, placing the basket in the extended position, maneuvering the basket to surround the material by moving at least one of the legs independently from at least one of the other legs, drawing all of the legs back into the lumen to grasp the material with the legs of the basket, and withdrawing the device from the body to remove the grasped material from the body.

The foregoing and other objects, aspects, features, and advantages of the invention will become more apparent from the following description and from the claims.

### Brief Description of the Drawings

In the drawings, like reference characters generally refer to the same parts throughout the different views. Also the drawings are not necessarily to scale, emphasis instead generally being placed upon illustrating the principles of the invention.
FIG. 1A is a plan view of a medical retrieval device with legs that extend along the entire length of the sheath to the actuating handle.
FIG. 1B is a plan view of a medical retrieval device with at least one elongate member in the lumen of the sheath.
FIG. 1C is an end view of a non-perforated tip of a basket in accordance with the invention.
FIG. 1D shows another basket of the invention formed by at least two loops, the loops being unattached at the point where the loops intersect at the distal end of the basket.
FIG. I E shows another basket of the invention similar to FIG. 1C where the basket is formed by a plurality of loops, the loops being unattached where the loops intersect at the distal end of the basket.
FIG. 2 is a plan view of one embodiment of a device according to the invention, with the basket of the device in an extended closed position and having one actuateable leg, two elongate members, and two lumens in the sheath.
FIG. 3 shows another basket which is similar to the basket of FIG. 2 with the basket in the extended open position.
FIG. 4A is a plan view of one embodiment of a basket according to the invention, with the basket in an extended closed position and having a pair of actuateable legs, two elongate members, and a single lumen within the sheath.
FIG. 4B shows the basket of FIG 4A in an extended open position.
FIG. 4C shows the basket of FIG. 4A in the sheath, where the sheath defines only a single lumen therewithin.
FIG. 4D is an end view of another embodiment of the invention showing a plurality of elongate members and lumens within the sheath.
FIGS. 5A-5E are diagrammatic representations of a clinical application of one embodiment of the device illustrated in FIGS. 4A, 4B, and 4C.
FIGS. 6A-6C show another embodiment of a medical retrieval device according to the invention, and the device in use.
FIG. 6D shows a three-dimensional view of a basket of the invention with the sheath removed where a second elongate member is attached to each basket leg.
FIG. 6E is an end view of the proximal portion of the basket illustrating different arrangements for the attachment site where one or more second elongate members attach to the proximal portion of the basket legs.

### Description

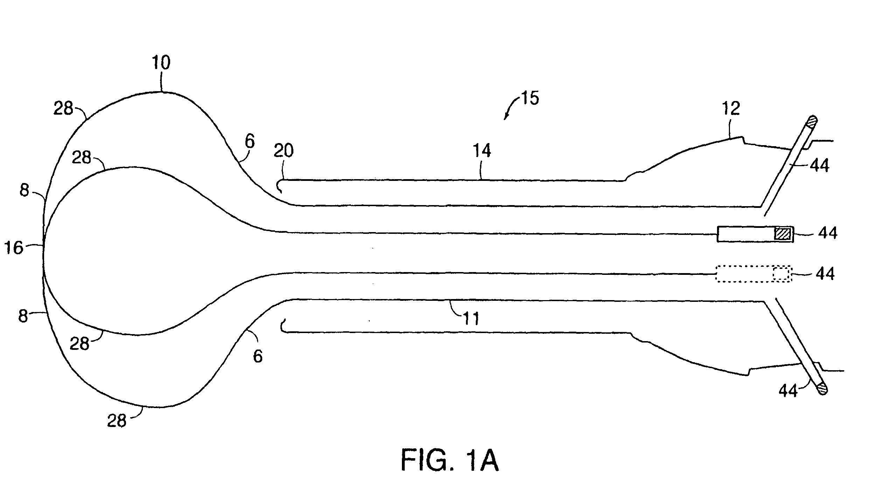

Referring to FIG. 1A, a medical retrieval device 15 for retrieving biological material or foreign material from a body comprises a distal basket 10, a proximal handle 12, and a sheath 14 disposed therebetween. The sheath 14 has at least one lumen 11 therewithin. The basket 10 is moveable relative to the sheath between a collapsed position and an extended position, and it is shown extended in a closed position in FIG. 1A. In one embodiment, the basket 10 comprises a non-perforated tip 16 and a plurality of legs 28. Each of the legs 28 having a distal end 8 at the tip of the basket 10 and a proximal end 6 at the base of the basket 10. The distal end 8 of each leg meets at the tip 16, and the proximal end 6 of each leg extends at least to a distal end 20 of the sheath 14.

In the disclosed embodiment, the basket legs are made of metal wire, although a variety of other materials can be used to form the legs such as polymers. The sheath 14 typically is made of a biocompatible material such as a plastic.

With continued reference to FIG. 1A, one or more lumens 11 extend within and are defined by the sheath 14. In some embodiments, there is one lumen 11, and the proximal end 6 of each of the legs 28 extends into that lumen 11. In one embodiment, each basket leg 28 runs individually down the entire length of the sheath 14. By keeping each leg 28 independent as it extends from the basket tip 16 to the actuating handle 12, several modes of operation can be achieved. For example, all basket legs can be actuated equally together, or an independent actuation of a leg can be performed. By actuating only one or two legs the basket can be maneuvered or tilted in a body tract, thereby increasing the ability to manipulate, capture or release a stone.

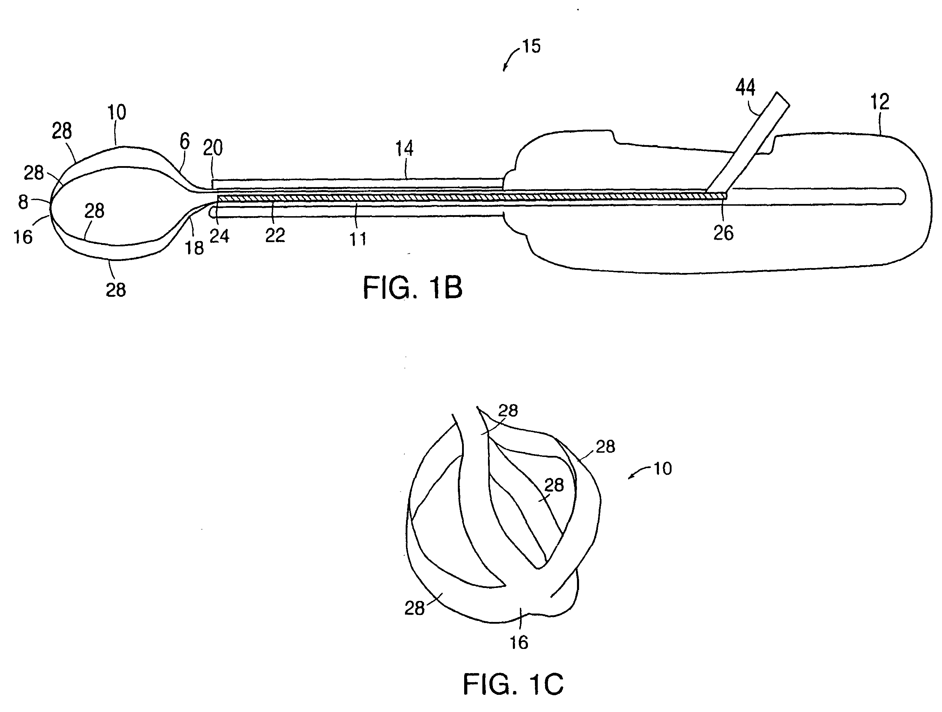

Referring to FIG. 1B in another embodiment of the invention, one or more elongate members 22 which are disposed in the lumen(s) 11 are operably attached to the proximal end 6 of one or more of the basket legs 28. The remaining legs may be attached to another elongate member or to a fixed point such as the end 20 of sheath 14. The elongate member(s) 22 are slideably moveable within the lumen 11 of the sheath 14, and they can be shafts, wires, coils, cables, or constructed in a variety of other ways. In the disclosed embodiment, the member(s) 22 generally are single-strand metal wires. The proximal end 26 of the elongate member 22 may be attached to an actuating device such as a slider 44 in the handle 12. Advancing or retracting the slider 44 advances or retracts one or more basket legs 28 attached to a distal end 24 of the elongate member(s) 22. The device 15 could alternately be constructed to achieve movement of the sheath 14 over the member(s) 22, but that is not shown.

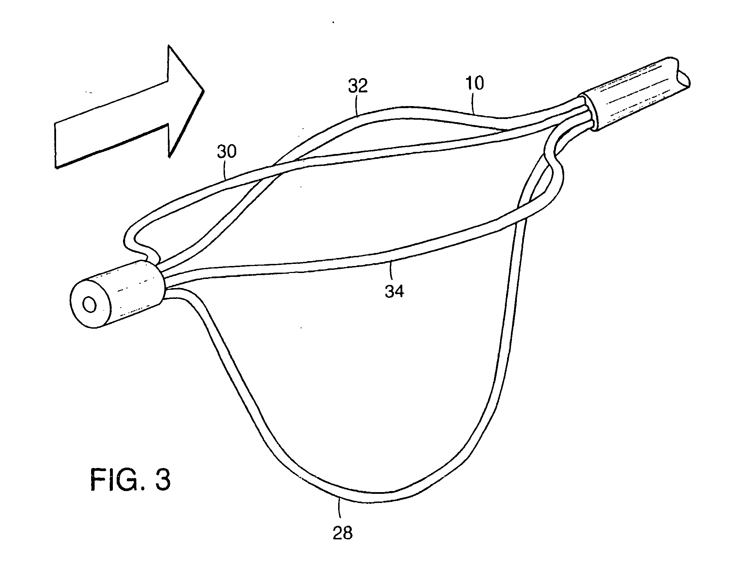

In a disclosed embodiment, the tip 16 is non-perforated as shown in FIG. 1C. That is, the tip 16 has no openings in it. Instead of being flat, as shown in FIG. 1C, the tip 16 of the basket can have a button, knob, or other protrusion, as shown for example in FIG. 3, which gathers the distal ends of the basket legs and holds them together.

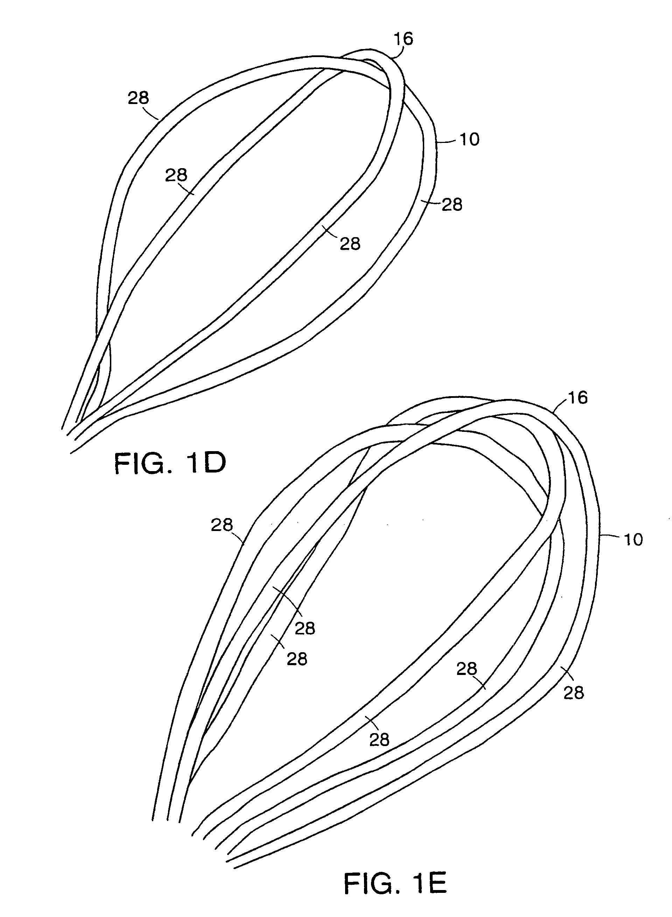

Referring to FIGs. 1D and 1E, in an alternative embodiment of the invention, the basket has two or more separate loops that intersect at the distal end of the basket. The loops are loose, that is the loops are not joined, secured or attached to each other at the distal end of the basket. The width of the space between the loops at the distal end of the basket is changeable when an unsecured loop at the distal end of the basket is extended.

While four-leg baskets are shown and described with reference to FIGS. 1A-Dand other drawings, it should be understood that any number of legs is possible. In general, three or more legs are required to form a basket as illustrated in FIG. 1E, three loops of the basket form a 6-leg basket.

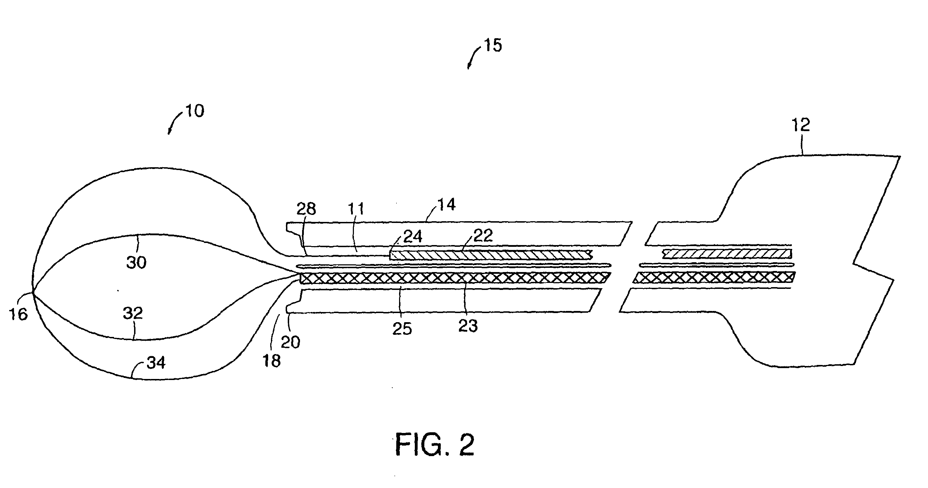

In one embodiment, as shown in FIG. 2 with the basket 10 in an extended, closed position, one of the legs (28) of the basket 10 is attached to the distal end 24 of a first elongate member 22 within a first lumen 11 of the sheath 14, and the three other legs (30, 32, 34) of the basket 10 are attached at the base 18 of basket 10 to a second elongate member 23 in a second lumen 25. Referring to FIG. 3, advancing the first elongate member 22 in a direction towards the distal end 20 of the sheath 14 causes the leg 28 to extend further from sheath 14 than the other basket legs 30, 32 and 34, thereby forcing leg 28 to bend outward and altering or tilting the shape of the expanded basket 10 into an extended, open position.

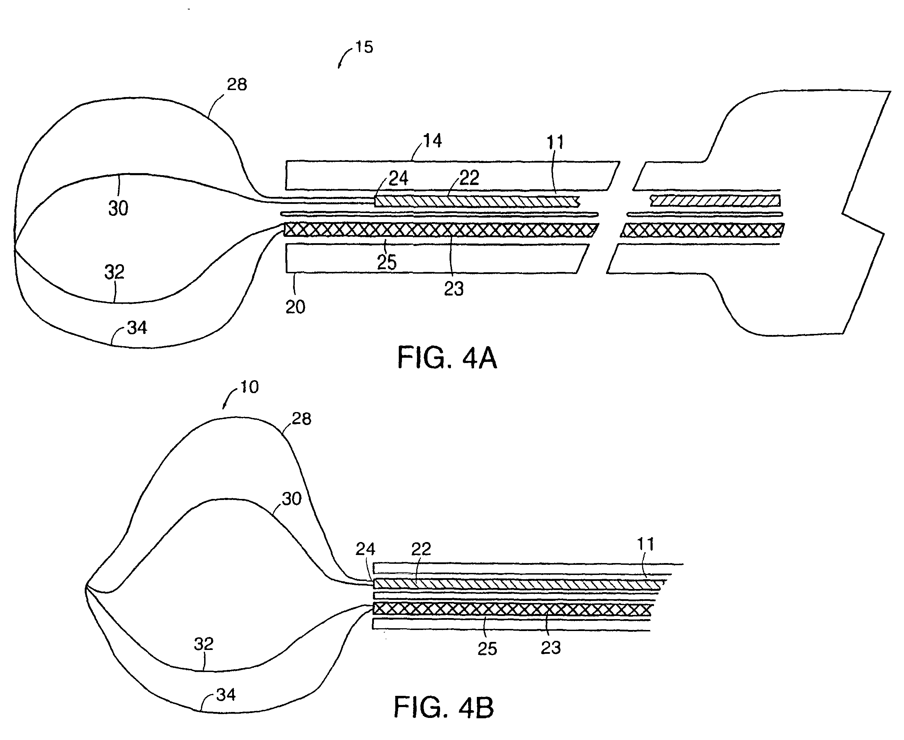

Referring to FIG. 4A, in another embodiment, two of the legs (28, 30) are attached to the distal end 24 of the first elongate member 22, and the two remaining legs (32, 34) are attached to the distal end of the second elongate member 23. The two elongate members 22 and 23 are enclosed within the first lumen 11 and the second lumen 25 of sheath 14, respectively. When the first elongate member 22 is advanced toward the distal end 20 of the sheath 14, the legs 28 and 30 hyperextend from the end of the sheath 14 and thereby cause the basket 10 to move and alter its symmetrical shape. In FIG. 4B, the basket 10 is shown with the legs 28, 30 hyperextended such that the basket 10 assumes a tilted shape in the extended, open position.

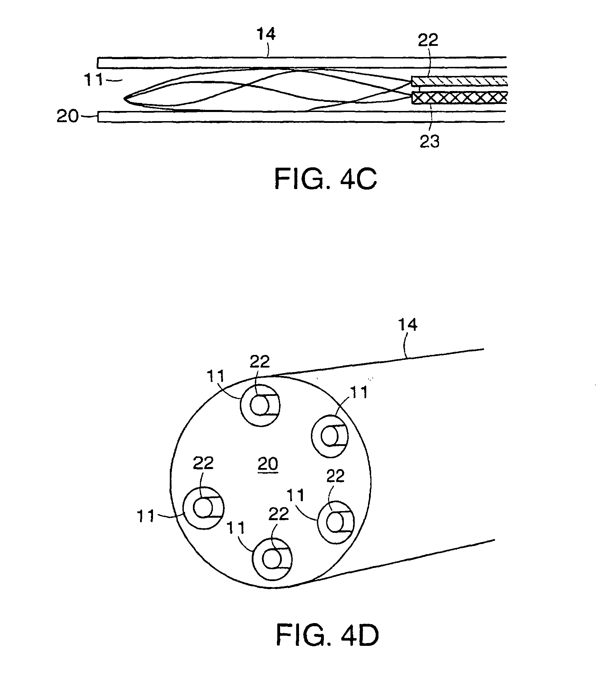

In another embodiment, each leg may be enclosed within a single, common axially disposed lumen 11 as illustrated in FIG. 4C. In this embodiment, the entire basket may be withdrawn into the single lumen with the basket in a collapsed position, as shown.

In other embodiments of the invention, various ones of the legs can be attached to certain ones of the elongate members. As illustrated in FIG. 4D, there may be as many elongate members and as many lumens as there are legs. A plurality of lumens 11 are provided in the embodiment of the sheath 14 shown in FIG. 4D, and each lumen 11 is designed to accommodate a different one of the elongate members 22. Whatever the combination of legs and elongate members, the combination will result in a device 15 having a basket 10 with one or more individually actuateable legs that allow the basket to be steered, tilted, and generally maneuvered into a variety of positions and configurations beyond simple extended and collapsed, and this maneuverability or danceability allows an operator of the basket to more easily capture stones and other material with the basket legs.

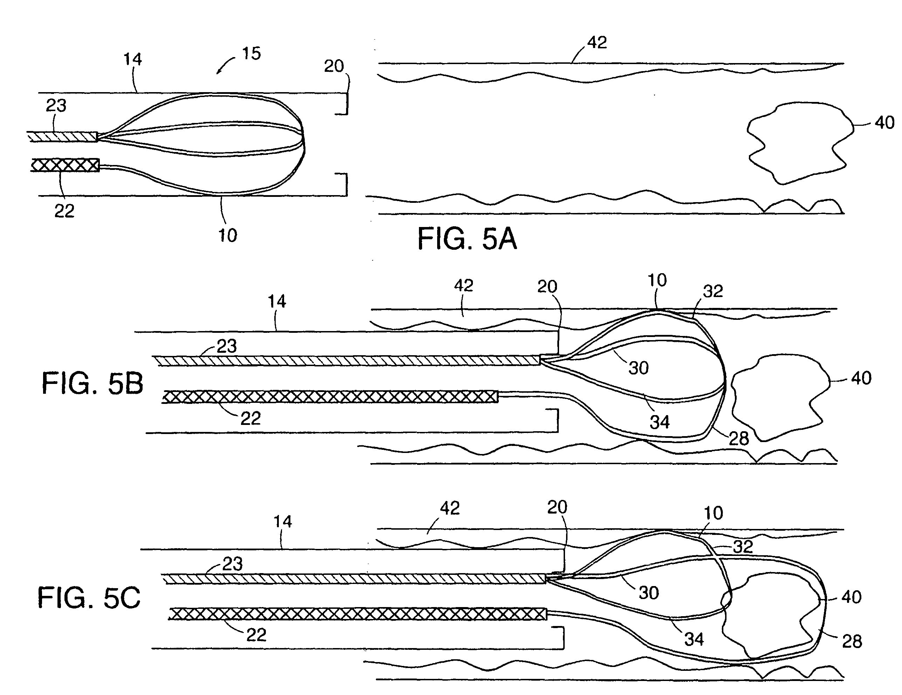

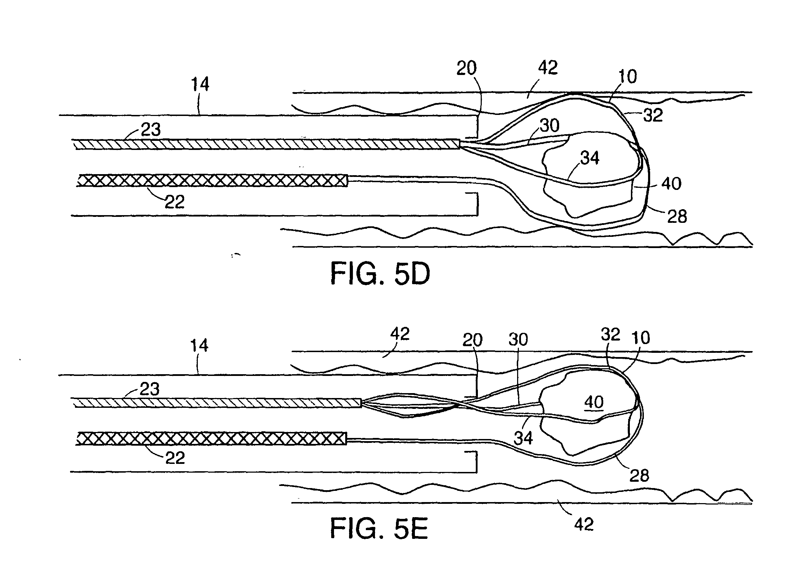

Referring to FIGS. 5A-5E, the device 15 of the invention can be used in a clinical application to retrieve biological material or foreign material from within a body. For example, the single-lumen device of FIG. 4C can be used to retrieve a stone 40 (e.g., a stone in the gall bladder, biliary tree, ureter, kidney, urinary bladder, urethra, etc.). It could also be used to capture a thrombus or embolus within a vessel such as the coronary vessels of the heart or the pulmonary vasculature. Regardless of what exactly is being retrieved, the device 15 with the basket 10 enclosed within the sheath 14 of the device is inserted into a tract 42 of the body. The tract 42 can be a canal, duct, tube, channel, vessel, or orifice of the body. As the distal end 20 of the sheath 14 approaches the stone 40 or other material, the basket 10 is deployed from the distal end 20 of the sheath 14 by advancing the slideably moveable elongate members 22 and 23 toward the distal end 20 of the sheath 14 (FIG. 5B). In another alternative embodiment, the sheath 14 may be withdrawn relative to the elongate members 22 and 23, thereby extending basket 10 from the end of the sheath 14.

After the basket is deployed to form a uniform three-dimensional basket shape (FIG. 5B) in the closed position, the single actuateable leg 28 is hyperextended by independently further advancing elongate member 22. The basket thus moves from the closed position shown in FIG. 5B to the open position illustrated in FIG. 5C. The distance between leg 28 and legs 30, 32, and 34 is thereby increased in size. The basket is thus maneuvered to ensnare the stone 40 by actuation of the leg 28. The individually-actuateable leg 28 is then withdrawn into the sheath 14 by independently retracting elongate member 22 (FIG. 5D) until the inner surface of the leg 28 and the inner surfaces of the other legs 30, 32, 34 are substantially in contact with the stone 40. The stone 40 can now be fully captured by the basket by further withdrawing the elongate members 22, 23 into the sheath 14 to draw all of the legs 28, 30, 32, 34 back towards the distal end of the sheath and around the stone 40. The device 15 with the stone 40 thus ensnared by the basket is now withdrawn from the tract 42 by the operator.

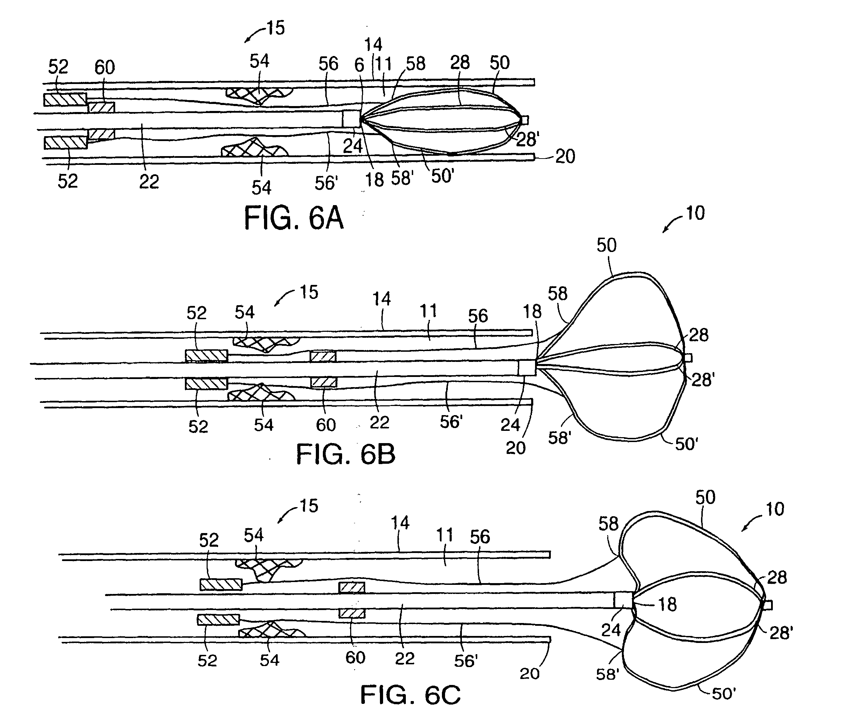

Other mechanisms for providing the individual leg actuatability in accordance with the invention are possible. Referring to FIGS. 6A-6E, the device 15 includes a slideably moveable ring 52 in the lumen 11 of sheath 14, a collar ring stop 54, a positive return ring stop 60, and a plurality of second elongate members 56, 56' operably attached to the legs 50-50' and the ring 52. Each of the basket legs 28, 28', 50, 50' are operably attached at their proximal ends 6 at the basket base 18 to the distal end 24 of a first elongate member 22 which is slideably moveable in lumen 1 1 of sheath 14. The second elongate member 56, 56' is operably attached at one end to the proximal portion 58, 58' of the legs 50, 50', respectively. The second elongate member 56, 56' extends Iongitudinally in the lumen 11 of the sheath 14 and is operably attached at its other end to the ring 52. The ring 52 encircles the first elongate member 22 within the lumen 11 of the sheath 14. The ring 52 is slideably moveable over the first elongate member 22 within the lumen 11. The collar ring stop 54, disposed on the inner surface of the sheath 14, stops the ring 52 from further axially advancing in the direction of the distal end 20 of the sheath 14. The positive return ring stop 60 attached to elongate member 22 is drawn into collar ring 52 as elongate member 22 is withdrawn into the lumen 11 in a direction opposite to distal end of the sheath 20.

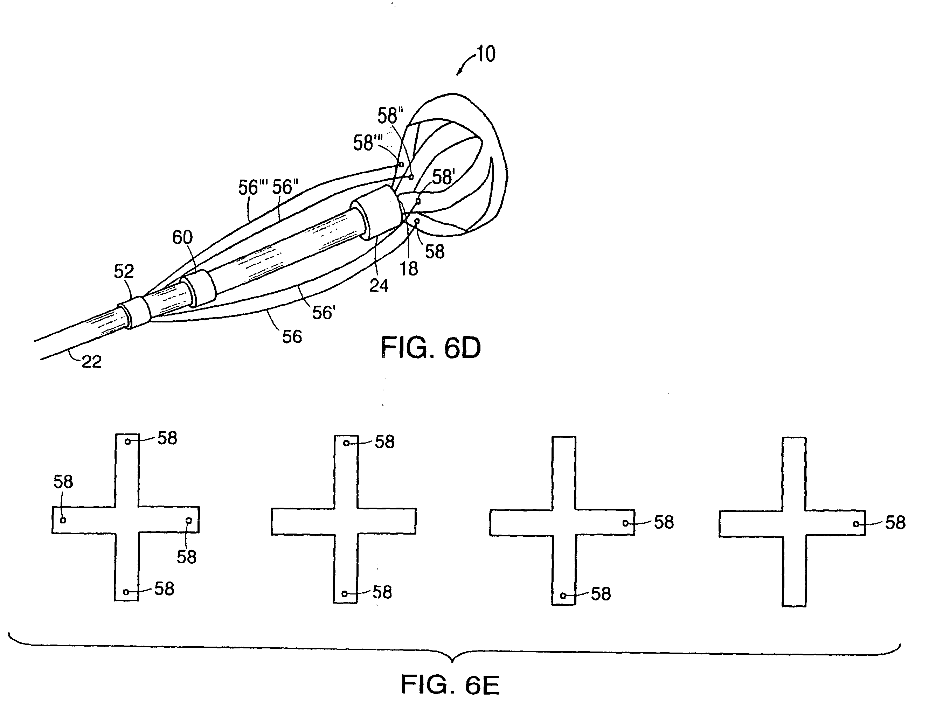

In other embodiments, second elongate members may be attached to one or more of the basket legs. For example, as illustrated in FIG. 6D, second elongate members 56, 56', 56" and 56''' are attached to each of the basket legs 58, 58', 58" and 58''', respectively. FIG. 6E illustrates different arrangements of the attachment 58 of second elongate members to one or more basket legs.

In operation, as shown in FIGS. 6B and 6C, as the first elongate member 22 is axially moved in the direction of the end 20 of the sheath 14, the basket 10 is advanced out of the sheath 14, simultaneously advancing the ring 52 in the lumen 11 of the sheath 14 until the ring 52 meets the collar ring stop 54. Further advancement of the ring 52 is prevented by the collar ring stop 54. As the first elongate member 22 is further axially advanced in the lumen 11, the second elongate member 56, 56' that is attached to the ring 52 and the legs 50, 50' prevents further axial advancement of the legs 50, 50'. The legs 50, 50' thus bow out as the first elongate member 22 further advances the legs 28, 28', thereby altering the shape of the open basket such that it takes on a type of mushroom profile.

Referring again to FIG. 6A, the basket 10 is withdrawn into the sheath 14 by withdrawing elongate member 22 in a direction away from the end 20 of the sheath 14. As elongate member 22 moves in the lumen 11 of sheath 14, positive ring stop 60 strikes collar ring 52. When positive ring stop 58 strikes collar ring 52, positive ring stop 60 moves collar ring 52 along with and in the same direction as elongate member 22 as elongate member 52 is withdrawn into the lumen 11 of the sheath 14.

In all embodiments according to the invention, the basket provides a dilatation force unachievable with traditional baskets. This is due to the extra force supplied by a user in moving one or more of the legs further than possible with known baskets. The extra force supplied by the operator's band gives rise to the basket's ability to provide an improved expansion force.

In another aspect of the invention, all or a part of the inner surface of one or more of the basket legs can be rough to enhance capture and retention of material such as stones and possibly also to crush or break material. The roughness of the inner surface(s) can be created by serrations, etching, teeth, or points on or in the inner surface(s). One or more of the basket legs can have such a rough inner surface.

## Claims

1. A medical retrieval device (15) comprising:
a proximal handle (12);
a sheath (14) extending from the handle (12) and including a lumen (11), the sheath (14) having a distal end (20) away from the handle (12);
at least one elongated member (22); and
a basket (10) comprising a distal end and a base (18), the basket (10) and the sheath (14) moveable relative to each other to achieve a collapsed position of the basket (10) in which the basket (10) is within the lumen (11) of the sheath (14) and restrained by said sheath (14); and an extended position of the basket (10) in which the basket (10) extends from the distal end (20) of the sheath (14) and assumes a three-dimensional shape out of the lumen (11) of the sheath (14), the basket (10) comprising at least three legs (28) joined at their distal end (8) to a distal end of the basket (10), each leg (28) fixedly attached to at least one elongated member (22), at least one of the legs (28) being moveable within the lumen (11) of said sheath (14) independently from at least one of the other legs (28).

2. The device of claim 1 wherein at least one of the legs (28) is coupled to a first elongate member (22) extending within the lumen (11) of the sheath (14) to the handle (12), and wherein at least one other different one of the legs (30, 32, 34) is coupled to a second elongate member (23) extending within the lumen (11) of the sheath (14) to the handle (12), such that independent movement of the first elongate member (22) within the sheath (14) causes independent movement of the leg (28) coupled thereto and independent movement of the second elongate member (23) within the sheath (14) causes independent movement of the leg (30, 32, 34) coupled thereto.

3. The device of claim 1 wherein at least one of the legs (28) includes an inner surface which has at least a portion that is rough.

4. The device of claim 1 wherein the distal end of the basket (10) is non-perforated.

5. The device of claim 1, wherein the elongated member is a first elongate member (22) disposed and moveable within the lumen (11) of the sheath (14) and attached at one end to the base (18) of the basket (10); and the device further comprises:
a ring (52) encircling the first elongate member (22) and disposed and moveable within the lumen (11) of the sheath (14);
a second elongate member (23) moveable within the lumen (11) of the sheath (14) and attached at one end to the ring (52) and at the other end to at least one of the legs (28); and
a stop (54) within the lumen of the sheath for contact by the ring (52) to prevent the second elongate member (23) from advancing beyond a predetermined distance within the lumen (11) such that further movement by the first elongate member (22) within the lumen (11) toward the distal end (20) of the sheath (14) causes the shape of the basket (10) to become altered.

6. The device of claim 1 further comprising at least two elongate members (22, 23) wherein at least one of the legs (28) is coupled to a different one of the at least two elongate members (22, 23) than at least one of the other legs (28), each elongate member (22, 23) disposed and moveable within a different lumen (11), and wherein at least one of the elongate members (22, 23) is moveable independently from at least one of the other elongate members (22, 23), such that independent movement of at least one of the elongate members (22, 23) within the sheath (14) causes independent movement of the at least one leg (28) coupled thereto.

7. The device of claim 1, wherein the sheath (14) defines only one lumen (11).

## Patentansprüche

1. Medizinische Herausholeinrichtung (15), die aufweist:
einen proximalen Griff (12);
einen Mantel (14), welcher sich vom Griff (12) aus erstreckt und ein Lumen (11) aufweist, wobei der Mantel (14) ein distales Ende (20) abgewandt vom Griff (12) hat;
zumindest ein langgestrecktes Teil (22); und
ein Fangkörbchen (10), welches aus einem distalen Ende und einer Basis (18) besteht, wobei das Fangkörbchen (10) und der Mantel (14) in bezug zueinander bewegbar sind, um eine zusammengeklappte Position des Körbchens (10) zu erreichen, bei der das Körbchen (10) innerhalb des Lumens (11) des Mantels (14) ist und durch den Mantel (14) zurückgehalten wird, und eine erweiterte Position des Körbchens (10), in welcher das Körbchen (10) sich vom distalen Ende (20) des Mantels (14) erstreckt und eine dreidimensionale Form außerhalb des Lumens (11) des Mantels (14) annimmt, wobei das Körbchen (10) zumindest drei Greifarme (28) aufweist, die an ihrem distalen Ende (8) mit einem distalen Ende des Körbchens (10) verbunden sind, wobei jeder Greifarm (28) fest an zumindest einem langgestreckten Teil (22) angebracht ist, wobei zumindest einer der Greifarme (28) innerhalb des Lumens (11) des Mantels (14) unabhängig von zumindest einem der anderen Greifarme (28) bewegbar ist.

2. Einrichtung nach Anspruch 1, wobei zumindest einer der Greifarme (28) mit einem ersten langgestreckten Teil (22) gekoppelt ist, welches sich innerhalb des Lumens (11) des Mantels (14) zum Griff (12) erstreckt, und wobei zumindest ein anderer verschiedener der Greifarme (30, 32, 34) mit einem zweiten langgestreckten Teil (23) gekoppelt ist, welches sich innerhalb des Lumens (11) des Mantels (14) zum Griff (12) erstreckt, so dass unabhängige Bewegung des ersten langgestreckten Teils (22) innerhalb des Mantels (14) unabhängige Bewegung des Greifarms (28), der daran gekoppelt ist, bewirkt und unabhängige Bewegung des zweiten langgestreckten Teils (23) innerhalb des Mantels (14) unabhängige Bewegung des Greifarms (30, 32, 34), der daran gekoppelt ist, bewirkt.

3. Einrichtung nach Anspruch 1, wobei zumindest einer der Greifarme (28) eine Innenfläche aufweist, die zumindest einen Bereich hat, der rau ist.

4. Einrichtung nach Anspruch 1, wobei das distale Ende des Fangkörbchens (10) nicht perforiert ist.

5. Einrichtung nach Anspruch 1, wobei das langgestreckte Teil ein erstes langgestrecktes Teil (22) ist, welches innerhalb des Lumens (11) des Mantels (14) angeordnet und bewegbar ist und an einem Ende der Basis (18) des Fangkörbchens (10) angebracht ist, und die Einrichtung außerdem aufweist:
einen Ring (52), der das erste langgestreckte Teil (22) umgibt und der innerhalb des Lumens (11) des Mantels (14) angeordnet und bewegbar ist;
ein zweites langgestrecktes Teil (22), welches innerhalb des Lumens (11) des Mantels (14) bewegbar ist und an einem Ende am Ring (52) und am anderen Ende an zumindest einem der Greifarme (28) angebracht ist; und
ein Halteteil (54) innerhalb des Lumens des Mantels zum Kontakt durch den Ring (52), um zu verhindern, dass das zweite langgestreckte Teil (23) sich über eine vorher festgelegte Distanz innerhalb des Lumens (11) weiterbewegt, so dass weitere Bewegung durch das erste langgestreckte Teil (22) innerhalb des Lumens (11) in Richtung auf das distale Ende (20) des Mantels (14) bewirkt, dass die Form des Fangkörbchens (10) geändert wird.

6. Einrichtung nach Anspruch 1, welche außerdem zumindest zwei langgestreckte Teile (22, 23) aufweist, wobei zumindest einer der Greifarme (28) mit einem anderen der zumindest beiden langgestreckten Teile (22, 23) als zumindest mit einem der anderen Greifarme (28) gekoppelt ist, wobei jedes langgestreckte Teil (22, 23) innerhalb eines unterschiedlichen Lumens (11) angeordnet und bewegbar ist und wobei zumindest eines der langgestreckten Teile (22, 23) unabhängig von zumindest einem der anderen langgestreckten Teile (22, 23) bewegbar ist, so dass unabhängige Bewegung von zumindest einem der langgestreckten Teile (22, 23) innerhalb des Mantels (14) unabhängige Bewegung des zumindest einen Greifarms (28), der daran gekoppelt ist, bewirkt.

7. Einrichtung nach Anspruch 1, wobei der Mantel (14) lediglich ein Lumen (11) begrenzt.

## Revendications

1. Dispositif de récupération médical (15), comprenant :
une poignée proximale (12) ;
une gaine (14) s'étendant à partir de la poignée (12) et ayant au moins un lumen (11, la gaine (14) ayant une extrémité distale (20) éloignée de la poignée (12) ;
au moins un élément allongé (22) ; et
un panier (10) comprenant une extrémité distale et une base (18), le panier (10) et la gaine (14) étant mobiles l'un par rapport à l'autre afin d'atteindre une position affaissée du panier (10) dans laquelle le panier (10) se trouve à l'intérieur du lumen (11) de la gaine (14) et est retenu par ladite gaine (14) ; et une position étendue du panier (10) dans laquelle le panier (10) s'étend à partir de l'extrémité distale (20) de la gaine (14) et prend une forme tridimensionnelle du lumen (11) de la gaine (14), le panier (10) comprenant au moins trois bras (28) joints au niveau de leur extrémité distale (8) à une extrémité distale du panier (10), chaque bras (28) étant fixement attaché à au moins un élément allongé (22), au moins l'un des bras (28) étant mobile à l'intérieur du lumen (11) de ladite gaine (14) indépendamment d'au moins l'un des autres bras (28).

2. Dispositif selon la revendication 1, dans lequel au moins l'un des bras (28) est couplé à un premier élément allongé (22) s'étendant à l'intérieur du lumen (11) de la gaine (14) vers la poignée (12), et dans lequel au moins un autre bras (30, 32, 34) différent est couplé à un second élément allongé (23) s'étendant à l'intérieur du lumen (11) de la gaine (14) vers la poignée (12), afin qu'un mouvement indépendant du premier élément allongé (22) à l'intérieur de la gaine (14) entraîne un mouvement indépendant du bras (28) couplé à celle-ci et qu'un mouvement indépendant du second élément allongé (23) à l'intérieur de la gaine (14) entraîne un mouvement indépendant du bras (30, 32, 34) couplé à celle-ci.

3. Dispositif selon la revendication 1, dans lequel au moins 1'un des bras (28) comprend une surface intérieure qui a au moins une partie qui est rugueuse.

4. Dispositif selon la revendication 1, dans lequel l'extrémité distale du panier (10) n'est pas perforée.

5. Dispositif selon la revendication 1, dans lequel l'élément allongé est un premier élément allongé (22) disposé et mobile à l'intérieur du lumen (11) de la gaine (14) et fixé à une extrémité de la base (18) du panier (10) ; et le dispositif comprend en outre :
une bague (52) encerclant le premier élément allongé (22) et disposée et mobile à l'intérieur du lumen (11) de la gaine (14) ;
un second élément allongé (23) mobile à l'intérieur du lumen (11) de la gaine (14) et fixé au niveau d'une extrémité à la bague (52) et au niveau de l'autre extrémité à au moins l'un des bras (28) ; et
une butée (54) à l'intérieur du lumen de la gaine pour être contactée par la bague (52) pour empêcher le second élément allongé (23) d'avancer au-delà d'une distance prédéterminée à l'intérieur du lumen (11) afin que tout autre mouvement effectué par le premier élément allongé (22) à l'intérieur du lumen (11) vers l'extrémité distale (20) de la gaine (14) entraîne une modification de la forme du panier (10).

6. Dispositif selon la revendication 1 comprenant en outre au moins deux éléments allongés (22, 23), dans lequel au moins l'un des bras (28) est couplé à un élément des au moins deux éléments allongés (22, 23) différent d'au moins l'un des autres bras (28), chaque élément allongé (22, 23) étant disposé et mobile à l'intérieur d'un lumen (11) différent, et dans lequel au moins l'un des éléments allongés (22, 23) est mobile indépendamment d'au moins l'un des autres éléments allongés (22, 23), afin que le mouvement indépendant d'au moins l'un des éléments allongés (22, 23) à l'intérieur de la gaine (14) entraîne un mouvement indépendant de l'au moins un bras (28) couplé à celui-ci.

7. Dispositif selon la revendication 1, dans lequel la gaine (14) définit un seul lumen (11).
